Europäisches Patentamt

European Patent Office

Office européen des brevets

(19)

(11) Veröffentlichungsnummer: **0 004 405**
**B1**

(12) **EUROPÄISCHE PATENTSCHRIFT**

(45) Veröffentlichungstag der Patentschrift:
25.11.81

(21) Anmeldenummer: **79200133.1**

(22) Anmeldetag: **16.03.79**

(30) Priorität: **20.03.78 DE 2812180**

(43) Veröffentlichungstag der Anmeldung:
**03.10.79 Patentblatt 79/20**

(45) Bekanntmachung des Hinweises auf die Patenterteilung:
**25.11.81 Patentblatt 81/47**

(84) Benannte Vertragsstaaten:
**BE CH DE FR GB IT LU NL SE**

(56) Entgegenhaltungen:
**GB-A-1 038 010**

(51) Int. Cl.³: **C 07 D 307/89**

(54) **Verfahren zur Herstellung von hochreinem Tetrahydrophthalsäureanhydrid.**

(73) Patentinhaber: **CHEMISCHE WERKE HÜLS AG,
Postfach 1320, D-4370 Marl 1 (DE)**

(72) Erfinder: **Gude, Fritz, Dr., Wilhelmstrasse 4,
D-4690 Herne 2 (DE)**
Erfinder: **Freitag, Karl-Ernst, Dr., Horster Strasse 528,
D-4250 Bottrop (DE)**

(74) Vertreter: **Steil, Hanna, Dipl.-Chem., RSP PATENTE -
PB 40 Herzogstrasse 28 Postfach 2840,
D-4690 Herne 2 (DE)**

## Verfahren zur Herstellung von hochreinem Tetrahydrophthalsäureanhydrid

Die Erfindung betrifft ein Verfahren zur Herstellung von Δ4-Tetrahydrophthalsäureanhydrid durch Reaktion von gasförmigem 1,3-Butadien und geschmolzenem Maleinsäureanhydrid, wobei ein Produkt höchster Reinheit erhalten wird.

Es gibt verschiedene Wege zur technischen Herstellung von Δ4-Tetrahydrophthalsäureanhydrid, die im Prinzip von 1,3-Butadien und Maleinsäureanhydrid ausgehen. Das Maleinsäureanhydrid kann in Lösung oder als Schmelze eingesetzt werden, das Molverhältnis der Komponenten kann variieren. Die Herstellungstemperaturen liegen im allgemeinen zwischen 40—140°C, der Druck variiert zwischen 1 und 10 bar.

Während der Synthese läßt es sich nicht vermeiden, daß unerwünschte Nebenprodukte entstehen. Sie treten um so stärker auf, je höher die Reaktionstemperaturen und das Molverhältnis zwischen Butadien und Maleinsäureanhydrid gewählt wird. Aus diesem Grunde hat man z. B. die Reaktion in einem geeigneten Lösungsmittel bei relativ niedriger Temperatur ausgeführt. Das Verfahren ist mit dem Nachteil behaftet, daß nach der Reaktion vom Lösungsmittel intensiv getrocknet werden muß. Ab 54°C ist es möglich, das Butadien in das geschmolzene Maleinsäureanhydrid einzuleiten, wobei man die Temperatur im Laufe der Reaktion mindestens bis auf etwa 103°C steigern muß, um das sich zunehmend bildende Δ4-Tetrahydrophthalsäureanhydrid sicher in der Schmelze zu halten. Dabei entstehen durch Nebenreaktionen zähe, klebrige Verunreinigungen (CH-PS 435 253).

Ein anderes Verfahren sieht vor, Maleinsäureanhydrid und Butadien in der Weise zu vereinigen, daß ständig ein Überschuß von Maleinsäureanhydrid vorhanden ist, um die Bildung von Polybutadien zu unterdrücken. Zusätzlich setzt man noch Polymerisationsinhibitoren, wie z. B. Hydrochinon und seine Homologen, Dichlorchinone oder p-tert.-Butylcatechol, zu. Zur Gewinnung des reinen Δ4-Tetrahydrophthalsäureanhydrids ist es nun nötig, das Reaktionsprodukt bei reduziertem Druck zu fraktionieren, wobei als Vorlauf das überschüssige Maleinsäureanhydrid abdestilliert.

Dieses umständliche Verfahren führt zwar zu einem reinen Endprodukt, ist aber besonders mit dem Nachteil behaftet, das Tetrahydrophthalsäureanhydrid im Vakuum destillieren zu müssen — wobei entweder ein sehr hohes Vakuum verwendet oder aber eine teilweise Verharzung des Endproduktes in der Blase in Kauf genommen werden muß, selbst wenn man unter Stickstoff arbeitet (CH-PS 489 478).

Nach einem weiteren Verfahren werden Maleinsäureanhydrid und Butadien in geschmolzenem Zustand oder in Lösungsmitteln im Molverhältnis 1 : 1 bis 1 : 1,3 umgesetzt, wobei die Reaktion in einer Füllkörperkolonne, die mit Raschig-Ringen gefüllt ist, erfolgt. Eine Reinigung erfolgt durch Umkristallisieren in Petroläther. Über Farbzahlen wurden keine Angaben gemacht (GB-PS 1 038 010).

Überraschenderweise wurde nun gefunden, daß man bei Anwendung von Butadien in stöchiometrischen Mengen bzw. in geringerem molekularen Überschuß auf sehr einfache und wirtschaftliche Weise ein hochreines, vollkommen farbloses Δ4-Tetrahydrophthalsäureanhydrid erhält, wenn man bestimmte Bedingungen einhält.

Gegenstand der Erfindung ist daher ein Verfahren zur Herstellung von hochreinem Δ4-Tetrahydrophthalsäureanhydrid aus geschmolzenem Maleinsäureanhydrid und gasförmigen 1,3-Butadien im Molverhältnis 1 : 1 bis 1 : 1,3 bei Temperaturen von 60—160°C und Drücken von 1—10 bar unter Rühren und anschließender Reinigung, welches dadurch gekennzeichnet ist, daß die anschließende Reinigung des geschmolzenen Produktes in einem poröse Füllkörper aus Keramik, Porzellan, Metall, Glas oder Kunststoff enthaltenden Wäscher in Gegenwart von Inertgas im Gegenstrom durchgeführt wird, wobei man überschüssiges Butadien über Kopf abtreibt und nach Passieren des rohen Tetrahydrophthalsäureanhydrids sämtliche polymere Verunreinigungen an den Füllkörpern haften bleiben.

Das erfindungsgemäße Verfahren führt zu reinstem Produkt mit Schmelzfarbzahlen von 10—20 Hazen. Auf den Füllkörpern, die aus porösem Material bestehen, scheiden sich überraschenderweise alle entstehenden Verunreinigungen ab, die sich aus Butadien oder Butadien und Maleinsäureanhydrid während oder nach der Synthese gebildet haben, als zähe, klebrige, offenbar vernetzte Harze ab, so daß das aus dem Wäscher austretende Δ4-Tetrahydrophthalsäureanhydrid rein und farblos ist.

Das Verfahren gemäß dieser Anmeldung bedarf also nur einfachster Handhabung, d. h. von Zeit zu Zeit müssen die Füllkörper der Wäscher gereinigt werden, z. B. mit organischen Lösungsmitteln, wobei sich Ketone besonders bewährt haben. Eine fraktionierte Destillation bei niedrigem Vakuum — wie oben genannt — ist entbehrlich.

Die Menge der bei dem Verfahren gemäß dieser Patentanmeldung anfallenden und in dem Wäscher quantitativ zurückgehaltenen Verunreinigungen ist relativ gering. Trotz der geringen Konzentration beeinflussen sie sehr stark die Farbe des Δ4-Tetrahydrophthalsäureanhydrids. Alkydharze, Polyester und andere Polymere zeigen bei Verwendung von diesen Verunreinigungen enthaltenen Tetrahydrophthalsäureanhydrid deutliche Trübungen, die seine Verwendung sehr einschränkt oder häufig sogar ausschließt.

Die Untersuchung der Verunreinigungen ergab, daß sich unter Vernetzung ein klebriges, Sauerstoff enthaltendes Polymerisat, bzw. Polymerisatgemisch gebildet hatte.

### Beispiel 1

In eine 60° C heiße, unter Stickstoff befindliche Schmelze von 6.500 Gewichtsteilen Maleinsäureanhydrid leitet man unter Rühren innerhalb von etwa 8 Stunden 3.620 Gewichtsteile entstabilisiertes, trockenes, gasförmiges Butadien (Molverhältnis 1 : 1,02) ein, wobei man mit fortschreitendem Umsatz die Temperatur der Schmelze allmählich bis zu einer Endtemperatur von 120° C anhebt. Es folgt eine 1/2stündige Nachreaktion bei 120° C.

Anschließend leitet man während 1—2 Stunden unter Rühren die 2 bis 3fache Volumenmenge des Ansatzes an trockenem Stickstoff in das Reaktionsgefäß, um überschüssiges Butadien weitgehend zu entfernen. Nun wird das Reaktionsprodukt in eine Füllkörperkolonne aus Keramik gepumpt, in der der herabfließenden Schmelze etwa das 2—3fache ihres Volumens an trockenem Stickstoff entgegenströmt. Durch diese Behandlung entfernt man das Butadien vollständig aus der Substanz und hält die klebrigen Verunreinigungen*) quantitativ zurück. Unter Inertgasatmosphäre wird dann die Schmelze auf einer Kühlwalze geschuppt.

Das fertige Produkt besitzt eine Schmelzfarbzahl von 10 Hz (ASTM D 2280/66) und ist frei von Butadien.

### Beispiel 2

### (Vergleichsbeispiel)

Die Verfahrensweise erfolgt analog dem oben beschriebenen Beispiel 1, jedoch wird abweichend davon das Produkt im Anschluß an die Nachreaktion nicht über die Füllkörperkolonne gepumpt.

Die Schuppen besitzen danach eine Schmelzfarbzahl von 50 Hz.

---

*) Eine Durchschnittsprobe der angefallenen, festen, an den Füllkörpern haftenden Substanz wurde mit Acetonitril erschöpfend extrahiert. Dadurch konnte 80,4 Gew.-% Tetrahydrophthalsäureanhydrid abgetrennt werden. Zurück blieb 19,6% klebriges, offenbar vernetztes, amorphes Material, das folgende Elementaranalyse aufwies:

C 61,15%; H 5,41%; O 33,1%.

Ansatz zur Herstellung eines Alkydharzes unter Verwendung des nach Beispiel 1 und 2 gewonnenen Δ4-Tetrahydrophthalsäureanhydrids

Gleiche molare Mengen von Tetrahydrophthalsäureanhydrid und Monoäthanolamin werden vereinigt und bei etwa 200° C ein Vakuum angelegt, um das gebildete Wasser zu entfernen.

Anschließend fügt man bei 150° C 1/6 Mol eines pflanzlichen Öles, z. B. Rizinusöl, zu.

Dabei fällt bei Verwendung von Tetrahydrophthalsäureanhydrid gemäß Beispiel 2 ein gelartiger Niederschlag aus, der nicht mehr in Lösung gebracht werden kann. Wegen Verstopfung des Filtermaterials ist eine Abtrennung durch Filtration kaum möglich.

Mit dem Tetrahydrophthalsäureanhydrid gemäß Beispiel 1 fällt dagegen bei gleichem Vorgehen keine Ausfällung an. Man kann durch Weiterreaktion mit den üblichen Reaktionspartnern ein einwandfreies, klares Alkydharz herstellen.

### Patentanspruch

Verfahren zur Herstellung von hochreinem Δ4-Tetrahydrophthalsäureanhydrid aus geschmolzenem Maleinsäureanhydrid und gasförmigem 1,3-Butadien im Molverhältnis 1 : 1 bis 1 : 1,3 bei Temperaturen von 60—160° C und Drücken von 1—10 bar unter Rühren und anschließender Reinigung, dadurch gekennzeichnet, daß die anschließende Reinigung des geschmolzenen Produktes in einem poröse Füllkörper aus Keramik, Porzellan, Metall, Glas oder Kunststoff enthaltenden Wäscher in Gegenwart von Inertgas im Gegenstrom durchgeführt wird, wobei man überschüssiges Butadien über Kopf abtreibt und nach Passieren des rohen Tetrahydrophthalsäureanhydrids sämtliche polymere Verunreinigungen an den Füllkörpern haften bleiben.

### Claim

Process for the production of very pure Δ4-tetrahydrophthalic acid anhydride from molten en maleic acid anhydride and gaseous 1,3-butadiene in a molar ratio of 1 : 1 to 1 : 1,3 at a temperature of 60—160° C and pressures of 1—10 bar under stirring and subsequent purification, comprising that the subsequent purification of the molten product is performed in a cleaner containing porous filling bodies of ceramics, porcelain, metal, glass or plastic in presence of inert gas in counterflow, whereby excess butadiene is removed overhead and after passing of the raw tetrahydrophthalic acid anhydride all polymeric impurities remain at the filling bodies.

**Revendication**

Procédé pour la fabrication d'anhydride Δ4-tétrahydrophthalique ultra pur à partir d'anhydride maléique fondu et de butadiène-1,3 gazeux, en proportion molaire de 1 : 1 à 1 : 1,3, à une température de 60—160°C et sous une pression de 1 à 10 bars, sous agitation et avec purification subséquente, procédé caractérisé en ce que la purification subséquente du produit fondu s'effectue dans un appareil de lavage contenant des corps de remplissage en céramique, porcelaine, métal, verre ou matière plastique, en présence d'un gaz inerte circulant à contre-courant, le butadiène en excès étant éliminé en tête de colonne, et après passage de l'anhydride tétrahydrophthalique brut, toutes les impuretés polymérisées restent adhérentes sur les corps de remplissage.